# EUROPÄISCHE PATENTANMELDUNG

(11) **EP 1 426 077 A1**
(43) Veröffentlichungstag der Anmeldung: **09.06.2004**
(21) Anmeldenummer: 03025835.4
(22) Anmeldetag: 11.11.2003
(51) Int. Cl.: A61M 39/28

(54) **Vorrichtung zur Regulierung der Strömung in Schlauchleitungen sowie enterales Überleitsystem mit einer derartigen Vorrichtung**

(30) Priorität: 05.12.2002 DE 10256772
(71) Anmelder: Fresenius Kabi Deutschland GmbH, 61352 Bad Homburg v.d.H. (DE)
(72) Erfinder: Magerl, Wolfgang, 56075 Koblenz (DE); Schäfer, Helmut, 61250 Usingen (DE)
(74) Vertreter: Luderschmidt, Schüler & Partner

(57) **Zusammenfassung**

Die Erfindung betrifft eine Vorrichtung zur Regulierung der Strömung in Schlauchleitungen für Infusions- und Transfusionsgeräte, insbesondere Schlauchleitungen für enterale Überleitsysteme. Die Vorrichtung verfügt über einen Basiskörper (2) und einen Torsionskörper (3), die jeweils ein Aufnahmestück (8, 12) aufweisen, in das ein Leitungsabschnitt der Schlauchleitung einlegbar ist. Das Aufnahmstück des Basiskörpers und das Aufnahmestück des Torsionskörpers sind auf einer Achse gegeneinander drehbar, so dass eine in den Aufnahmestücken liegende Schlauchleitung zur Reduzierung des Querschnitts tordiert werden kann. Darüber hinaus bezieht sich die Erfindung auf ein Überleitsystem zur enteralen Ernährung mit einer derartigen Vorrichtung. Der Vorteil der Vorrichtung liegt darin, dass sie von der Schlauchleitung jederzeit abgenommen oder an die Leitung angebracht werden kann. Darüber hinaus ist eine exakte Regulierung der Förderrate möglich.

## Beschreibung

Die Erfindung bezieht sich auf eine Vorrichtung zur Regulierung der Strömung in Schlauchleitungen für Infusions- und Transfusionsgeräte, insbesondere Schlauchleitungen für enterale Überleitsysteme. Darüber hinaus betrifft die Erfindung ein enterales Überleitsystem mit einer derartigen Vorrichtung.

Zur enteralen Ernährung sind Anordnungen bekannt, die einen die Nährlösung enthaltenden Behälter, ein an den Behälter angeschlossenes Überleitsystem und eine Sonde umfassen, über die dem Patienten die Nährlösung in den Magen appliziert wird. Die Förderung der Nährlösung kann entweder durch die Schwerkraft oder durch eine Pumpe erfolgen, in die das Überleitsystem eingelegt wird. Daher sind derartige Anordnungen als sogenannte Schwerkraftsets bzw. Pumpensets bekannt.

Zur Unterbrechung und Regulierung des Flüssigkeitsflusses finden bei den bekannten Schwerkraftsets Rollenklemmen Verwendung. Eine Rollenklemme ist beispielsweise aus der DE-A-196 21 910 bekannt. Bei Pumpensets wird der Flüssigkeitsfluß durch die Förderrate der Pumpe bestimmt.

Nachteilig ist, dass sich die bekannten Rollenklemmen nicht nachträglich an den Schlauchleitungen anbringen oder von den Leitungen abnehmen lassen. Im Übrigen hat sich in der Praxis gezeigt, dass eine genaue Einstellung der Förderrate mittels der bekannten Rollenklemmen insbesondere bei unterschiedlichen Sondennahrungen nicht immer möglich ist. Auch kann insbesondere bei Sondennahrungen, die eine starke Tendenz zu Okklusionen an Querschnittsverengungen zeigen, nicht immer die Stabilität der Flussrate gewährleistet werden.

Der Erfindung liegt daher die Aufgabe zugrunde, eine Vorrichtung zur Regulierung der Strömung in Schlauchleitungen für Infusions- und Transfusionsgeräte, insbesondere Schlauchleitungen für enterale Überleitsysteme zu schaffen, die einerseits eine exakte Regulierung der Förderrate bei unterschiedlichen Medien, insbesondere Sondennahrungen, erlaubt und andererseits nachträglich an die Schlauchleitungen angebracht oder von den Leitungen abgenommen werden kann. Eine weitere Aufgabe der Erfindung liegt darin, ein universell einsetzbares Überleitsystem zur enteralen Ernährung bereitzustellen, dessen Förderrate sich exakt regulieren lässt.

Die Lösung dieser Aufgaben erfolgt erfindungsgemäß mit den in den Patentansprüchen 1 bzw. 15 angegebenen Merkmalen. Vorteilhafte Ausführungsformen sind Gegenstand der Unteransprüche.

Bei der erfindungsgemäßen Vorrichtung erfolgt die Regulierung der Förderrate nicht durch Zusammendrücken sondern Verdrehen der Schlauchleitung. Eine Torsion der Schlauchleitung führt zu einer Querschnittsverringerung, die mit einer geringeren Förderrate verbunden ist.

Die Vorrichtung verfügt über einen Basiskörper und einen Torsionskörper, die jeweils ein Aufnahmestück aufweisen, in das ein Leitungsabschnitt der Schlauchleitung eingelegt werden kann. Das Aufnahmestück des Basiskörpers und das Aufnahmestück des Torsionskörpers sind auf einer gemeinsamen Achse gegeneinander verdrehbar, so dass die in den Aufnahmestücken liegende Schlauchleitung zur Reduzierung des Querschnitts tordierbar ist.

Ein wesentlicher Vorteil liegt darin, dass die Vorrichtung nachträglich an der Schlauchleitung angebracht werden kann. Dies ist beispielsweise dann von Vorteil, wenn eine Pumpenapplikation durch eine Schwerkraftapplikation substituiert werden soll, insbesondere zur Notfall-Regulierung nach Ausfall der Pumpe. Darüber hinaus ist vorteilhaft, dass die Vorrichtung jederzeit von der Schlauchleitung abgenommen werden kann, beispielsweise wenn sie während der Pumpenapplikation nicht gebraucht wird. Auch kann die Vorrichtung mehrfach verwandt werden. Darüber hinaus erlaubt die Vorrichtung eine exakte Regulierung der Förderrate auch bei unterschiedlichen Medien, insbesondere Sondennahrungen, wobei die Stabilität der Flussrate sichergestellt ist.

Die Aufnahmestücke des Basis- und Torsionskörpers können unterschiedlich ausgebildet sein. Es kommt allein darauf an, dass sich die Schlauchleitung in die Aufnahmestücke einlegen lässt. Vorzugsweise wird der Leitungsabschnitt in den Aufnahmestücken klemmend gehalten.

Bei einer bevorzugten Ausführungsform weisen die Aufnahmestücke eine durch zwei seitliche Schenkel begrenzte Nut auf, in die die Schlauchleitung eingelegt wird. Die Schenkel weisen vorteilhafterweise gekrümmte Anlageflächen auf, deren Querschnittsfläche bzw. Krümmungsradius geringfügig kleiner als der Leitungsquerschnitt bzw. -Radius der Schlauchleitung ist. Dadurch ist eine sichere Anlage gewährleistet. Alternativ können die Aufnahmestücke aber auch in der Art eines Schnappverschlusses für die Schlauchleitung ausgebildet sein, die zwischen einem oberen und unteren Bügel klemmend gehalten wird.

Bei einer bevorzugten Ausführungsform schließt sich an das Aufnahmestück des Basiskörpers ein Fixierungsstück mit einer durch zwei seitliche Schenkel begrenzten Nut auf, in der das Aufnahmestück des Torsionskörpers drehbar gelagert ist. Vorzugsweise ist der Torsionskörper in der Nut des Fixierungsstücks klemmend fixiert, so dass sich der Torsionskörper im Fixierungsstück nicht in Folge der Torsionskräfte der Schlauchleitung verdrehen kann. Diese Arretierung erweist sich als einfach aber sicher.

Eine weitere bevorzugte Ausführungsform sieht vor, dass das Aufnahmestück des Torsionskörpers einen ersten Abschnitt aufweist, der drehbar in die Nut des Fixierungsstücks eingesetzt ist und einen zweiten Abschnitt aufweist, der sich an der Stirnseite des Fixierungsstücks abstützt. Dadurch ist der Torsionskörper in einer Richtung gegen axiales Verschieben in dem Fixierungsstück gesichert.

Bei einer weiteren bevorzugten Ausführungsform weist das Aufnahmestück des Torsionskörpers einen vorspringenden Ansatz auf, der in einen seitlichen Schlitz des Fixierungsstücks eingesetzt ist. Vorzugsweise ist der Ansatz des Torsionskörpers als Stellrad ausgebildet, mit dem der Torsionskörper zur Regulierung der Strömung mit Daumen und Zeigefinger einfach in dem Fixierungsstück des Basiskörpers verdreht werden kann. Es ist aber auch möglich, den sich an der Stirnseite des Fixierungsstücks abstützenden zweiten Abschnitt des Aufnahmestücks als Stellrad auszubilden.

Zur sicheren Arretierung des Aufnahmestücks des Torsionskörpers in dem Fixierungsstück des Basiskörpers sind relativ hohe Klemmkräfte erforderlich. Daher ist das Verdrehen des Torsionskörpers relativ schwierig. Der Griffteil des Basiskörpers weist vorzugsweise zwei parallele Schenkel auf, die derart an dem Fixierungsstück angesetzt sind, dass durch Zusammendrücken der Schenkel des Griffteils die Schenkel des Fixierungsstücks auseinander spreizbar sind. Da mit Daumen und Zeigefinger Druck auf die beiden Schenkel des Griffteils ausgeübt werden kann, ist der Torsionskörper leichter drehbar. Dadurch wird die Handhabung weiter vereinfacht.

Die erfindungsgemäße Vorrichtung kann aus unterschiedlichen Materialien gefertigt sein. Vorzugsweise sind der Basis- und Torsionskörper Spritzgießteile aus Kunststoff.

Das Überleitsystem zur enteralen Ernährung gemäß der Erfindung weist eine Schlauchleitung auf, mit der eine Nährlösung aus einem Behälter über eine Sonde dem Patient zugeführt wird. Zum Anschluß des Behälters und der Sonde verfügt das Überleitsystem über die bekannten Konnektoren. Darüber hinaus umfasst das Überleitsystem die erfindungsgemäße Vorrichtung zur Regulierung der Strömung in der Schlauchleitung. Die Vorrichtung kann dem Überleitsystem beiliegen oder an der Schlauchleitung angebracht sein.

Im Folgenden wird ein Ausführungsbeispiel der Erfindung unter Bezugnahme auf die Zeichnungen näher erläutert.

Es zeigen:
- Fig. 1: eine Vorrichtung zur Regulierung der Strömung in Schlauchleitungen für enterale Überleitsysteme in perspektivischer Darstellung,
- Fig. 2: eine perspektivische Darstellung des Basiskörpers der Vorrichtung von Figur 1,
- Fig. 3: eine perspektivische Darstellung des Torsionskörpers der Vorrichtung von Figur 1,
- Fig. 4: eine vereinfachte Darstellung einer Anordnung zur enteralen Ernährung mit einem Überleitsystem und der Vorrichtung von Figur 1 und
- Fig. 5: eine alternative Ausführungsform des Torsionskörpers für die Vorrichtung von Fig. 1.

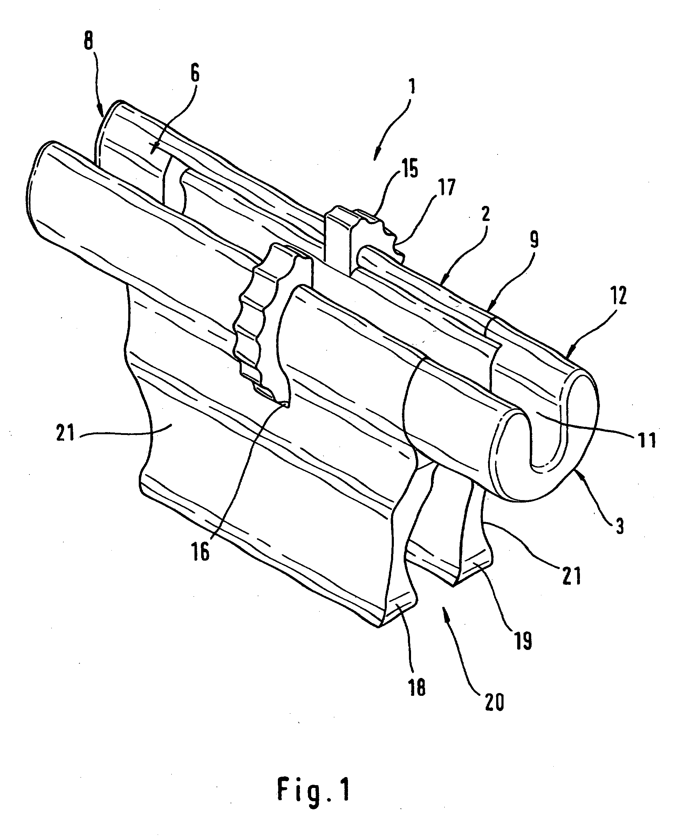

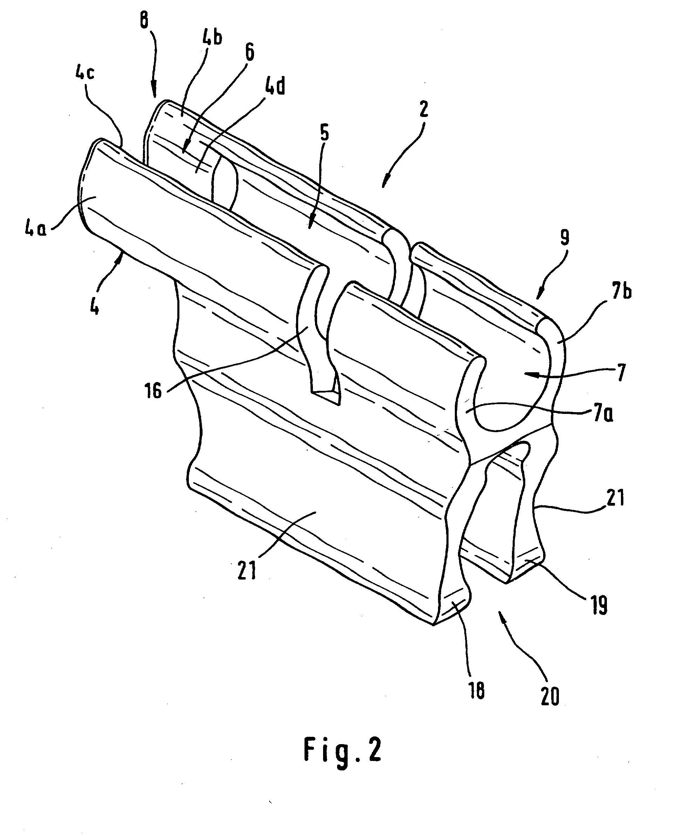

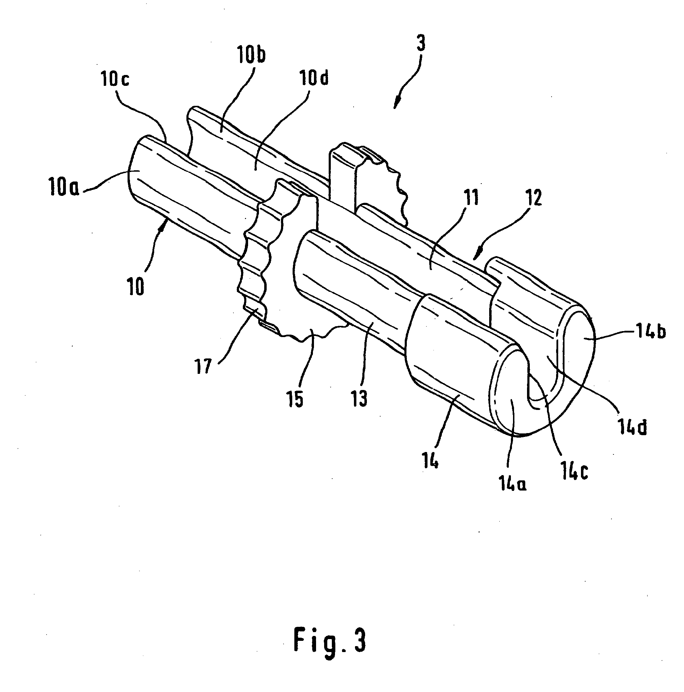

Die Vorrichtung 1 zur Regulierung der Strömung in der Schlauchleitung des Überleitsystems besteht aus einem Basiskörper 2 und einem Torsionskörper 3, die gegeneinander verdrehbar sind. Figur 1 zeigt eine perspektivische Darstellung der gesamten Vorrichtung, während die Figuren 2 und 3 in perspektivischer Darstellung den Basis- bzw. Torsionskörper 2, 3 zeigen.

Basis- und Torsionskörper 2, 3 sind Spritzgießteile aus Kunststoff. Der Basiskörper 2 umfasst einen stabförmigen Körper 4 mit einer längslaufenden Nut 5, die einen kürzeren Abschnitt 6 mit einer kleineren Querschnittsfläche und einen längeren Abschnitt 7 mit einer größeren Querschnittsfläche aufweist.

Der kürzere Abschnitt 6 der Nut 5 des stabförmigen Körpers 4 bildet ein Aufnahmestück 8 mit zwei seitlichen Schenkeln 4a, 4b, die gekrümmte Anlageflächen 4c, 4d aufweisen. Die Querschnittsfläche des kürzeren Abschnitts 6 der Nut 5 bzw. der Krümmungsradius der Schenkel 4a, 4b ist geringfügig kleiner als der Querschnitt bzw. Radius der Schlauchleitung, so dass die Schlauchleitung in dem Aufnahmestück 8 klemmend fixiert werden kann. Der längere Abschnitt 7 der Nut 5 des stabförmigen Körpers 4 bildet ein Fixierungsstück 9 für den Torsionskörper 3.

Der Torsionskörper 3 weist einen stabförmigen Körper 10 mit einer längslaufenden Nut 11 auf, der ebenfalls ein Aufnahmestück 12 für die Schlauchleitung bildet. Er weist einen längeren Abschnitt 13 mit einem kleineren Außendurchmesser und einen kürzeren Abschnitt 14 mit einem vorzugsweise größeren Durchmesser auf, wobei der kleinere Durchmesser geringfügig größer als der Querschnitt des längeren Abschnitts 6 der Nut 5 des Fixierungsstücks 9 des Basiskörpers 2 ist, so dass der Torsionskörper 3 in dem Fixierungsstück zwar drehbar gelagert ist, aber dennoch klemmend gehalten wird. Mit dem kürzeren Abschnitt 14 stützt sich der stabförmige Körper 10 des Torsionskörpers 3 an der Stirnseite des Fixierungsstücks 9 des Basiskörpers 2 ab.

Der Abschnitt der längslaufenden Nut 11 in dem längeren Abschnitt 13 des Aufnahmestücks 12 hat eine Querschnittsfläche, die dem Querschnitt der Schlauchleitung entspricht, so dass die Schlauchleitung lose in diesen Abschnitt der Nut eingelegt werden kann. Die beiden Schenkel 10a, 10b des stabförmigen Körpers 10 haben entsprechend gekrümmte Anlageflächen 10c, 10d. Der Abschnitt der Nut 11 in dem kürzeren Abschnitt 14 des Aufnahmestücks hingegen hat eine Querschnittsfläche, die geringfügig kleiner als der Querschnitt der Schlauchleitung ist, so dass die Leitung in diesem Abschnitt der Nut 11 zwischen den gekrümmten Anlageflächen 14c, 14d, der Schenkel 14a, 14b klemmend fixiert ist.

An seiner Aussenseite weist der stabförmige Körper 10 einen vorspringenden Ansatz 15 auf, der in einem seitlichen Schlitz 16 des Fixierungsstücks passend eingesetzt ist. Der Ansatz 15 ist als Stellrad ausgebildet, das eine Rändelung 17 aufweist.

An die beiden Schenkel 4a, 4b des Basiskörpers 2 schließen sich jeweils ein paralleler Schenkel 18, 19 an, die den Griffteil 20 der Vorrichtung bilden. Die Schenkel 18, 19 sind mit entsprechenden Griffmulden 21 versehen. Durch Zusammendrücken der beiden Schenkel 18, 19 des Griffteils 20 werden die Schenkel 7a, 7b des Fixierungsstücks 9 auseinandergespreizt, so dass der Torsionskörper 3 in dem Basiskörper 2 an dem Stellrad 15 leicht verdreht werden kann. Ansonsten ist der Torsionskörper in dem Basiskörper klemmend fixiert.

Die nicht dargestellte Schlauchleitung wird in die Nut 6 des Aufnahmestücks 8 des Basiskörpers 2 und die sich daran unmittelbar anschließende Nut 11 des Torsionskörpers 3 in der vorliegenden Darstellung von oben eingelegt. Die Querschnittsflächen sind dabei so bemessen, dass die Vorrichtung leicht klemmend auf der Schlauchleitung sitzt. Durch axiales Verdrehen des Torsionskörpers 3 mittels des Stellrades 15 gegenüber dem Basiskörper 2 wird der in dem Aufnahmestück 8 liegende Leitungsabschnitt gegenüber dem Leitungsabschnitt der Schlauchleitung verdreht, der in dem Aufnahmestück 12 liegt. Mit zunehmendem Drehwinkel wird die Querschnittsfläche der Schlauchleitung somit kontinuierlich verringert, so dass die Flussrate kleiner wird.

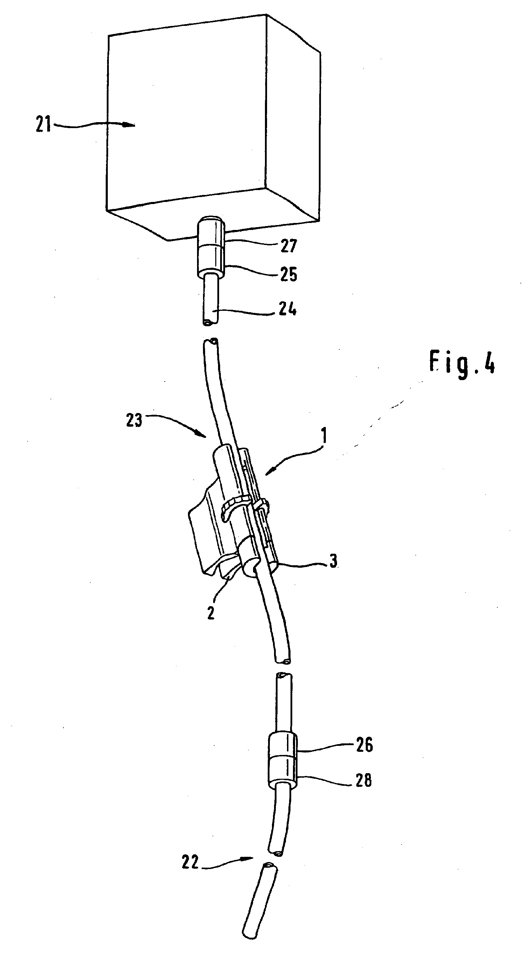

Figur 4 zeigt eine Darstellung einer Anordnung zur enteralen Ernährung, bei der die Nährlösung durch Schwerkraft gefördert wird. Diese Anordnung wird daher auch als Schwerkraftset bezeichnet. Das Schwerkraftset umfasst einen Behälter 21 zur Aufnahme der enteralen Nährlösung, eine Sonde 22 zum Zuführen der Nährlösung in den Magen des Patienten und ein Überleitsystem 23 zum Überführen der Nährlösung aus dem Behälter 21 in die Sonde 22. Das Überleitsystem 23 weist eine Schlauchleitung 24 auf, die an ihren Enden Konnektoren 25, 26 hat, mit denen die Leitung an die entsprechenden Verbindungsstücke 27, 28 des Behälters 21 bzw. der Sonde 22 angeschlossen wird.

Die Vorrichtung 1 zur Regulierung der Förderrate sitzt auf der Schlauchleitung 23. Durch Verdrehen des Torsionskörpers 3 gegenüber dem Basiskörper 2 kann die Förderrate exakt eingestellt werden. Wenn die Förderung der Nährlösung durch eine Pumpe erfolgen soll, kann die Vorrichtung 1 abgenommen werden. Wird die Nährlösung durch eine Pumpe gefördert, kann wiederum bei Ausfall der Pumpe die Vorrichtung 1 an der Schlauchleitung angebracht werden, so dass ein Notfallbetrieb möglich ist. Insofern erlaubt die Vorrichtung einen universellen Einsatz der bekannten Anordnungen zur enteralen Ernährung.

Bei dem unter Bezugnahme auf die Figuren 1 bis 4 beschriebenen Ausführungsbeispiel wird die Schlauchleitung in die Nuten der Aufnahmestücke gedrückt, was in der Praxis am einfachsten durch beidseitiges Ziehen am Schlauch erfolgt. Dies kann aber unter Umständen eine nicht unerhebliche Veränderung des Schlauchquerschnitts und somit der Förderrate zur Folge haben.

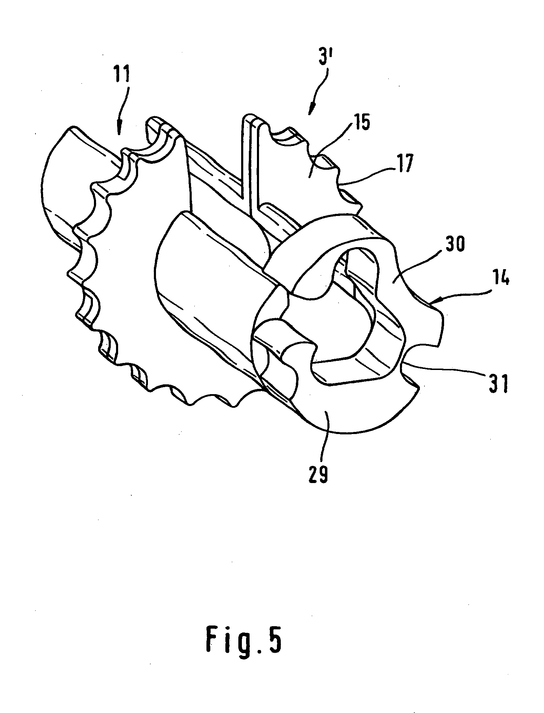

Fig. 5 zeigt eine alternative Ausführungsform des Torsionskörpers für die Vorrichtung von Fig. 1, bei der die Schlauchleitung zum Einlegen derselben nicht in die Länge gezogen werden muß. Die einander entsprechenden Teile sind mit den gleichen Bezugszeichen versehen. Der Torsionskörper 3' unterscheidet sich von dem obigen Ausführungsbeispiel dadurch, dass der äußere Abschnitt 14 des Aufnahmestücks 12 als Klemmteil ausgebildet ist. Das Klemmteil weist einen unteren und oberen Bügel 29, 30 auf, die über ein Filmscharnier 31 miteinander verbunden sind und einen Schnappverschluß für die Schlauchleitung bilden. Die Leitung wird zwischen die Bügel lose eingelegt und durch Zusammendrücken der Bügel klemmend festgelegt. Das Aufnahmestück 8 des Basiskörpers 2 kann ebenfalls als Klemmteil ausgebildet sein oder nur einer der beiden Aufnahmestücke.

## Patentansprüche

1. Vorrichtung zur Regulierung der Strömung in Schlauchleitungen für Infusions- und Transfusionsgeräte, insbesondere Schlauchleitungen für enterale Überleitsysteme, die einen Basiskörper (2) mit einem Aufnahmestück (8) aufweist, in das ein Leitungsabschnitt der Schlauchleitung einlegbar ist, **dadurch gekennzeichnet, dass** ein Torsionskörper (3) mit einem Aufnahmestück (12) vorgesehen ist, in das ein Leitungsabschnitt der Schlauchleitung einlegbar ist, wobei das Aufnahmestück (8) des Basiskörpers (2) und das Aufnahmestück (12) des Torsionskörpers (3) auf einer Achse gegeneinander drehbar sind, so dass eine in den Aufnahmestücken liegende Schlauchleitung zur Reduzierung des Querschnitts tordierbar ist.

2. Vorrichtung nach Anspruch 1, **dadurch gekennzeichnet, dass** das Aufnahmestück (8) des Basiskörpers (2) eine durch zwei seitliche Schenkel (4a, 4b) begrenzte Nut (6) aufweist, in die der Leitungsabschnitt der Schlauchleitung einlegbar ist.

3. Vorrichtung nach Anspruch 2, **dadurch gekennzeichnet, dass** die Schenkel (4a, 4b) des Aufnahmestücks (8) des Basiskörpers (2) gekrümmte Anlagenflächen (4c, 4d) für die Schlauchleitung aufweist.

4. Vorrichtung nach einem der Ansprüche 1 bis 3, **dadurch gekennzeichnet, dass** das Aufnahmestück (12) des Torsionskörpers (3) eine durch zwei seitliche Schenkel (10a, 10b; 14a, 14b) begrenzte Nut (11) aufweist, in die der Leitungsabschnitt der Schlauchleitung einlegbar ist.

5. Vorrichtung nach Anspruch 4, **dadurch gekennzeichnet, dass** die Schenkel (10a, 10b; 14a, 14b) des Aufnahmestücks (12) des Torsionskörpers (3) gekrümmte Anlageflächen (10c, 10d; 14c, 14d) für die Schlauchleitung aufweist.

6. Vorrichtung nach einem der Ansprüche 1 bis 5, **dadurch gekennzeichnet, dass** der Basiskörper (2) ein an dessen Aufnahmestück (8) anschließendes Fixierungsstück (9) mit einer durch zwei seitliche Schenkel (7a, 7b) begrenzten Nut (7) aufweist, in der das Aufnahmestück (12) des Torsionskörpers (3) drehbar gelagert ist.

7. Vorrichtung nach Anspruch 6, **dadurch gekennzeichnet, dass** das Aufnahmestück (12) des Torsionskörpers (3) in der Nut (7) des Fixierungsstücks (9) zwischen dessen Schenkeln (7a, 7b) klemmend fixiert ist.

8. Vorrichtung nach Anspruch 6 oder 7, **dadurch gekennzeichnet, dass** das Aufnahmestück (12) des Torsionskörpers (3) einen ersten Abschnitt (13), der drehbar in die Nut (7) des Fixierungsstücks (9) eingesetzt ist, und einen zweiten Abschnitt (14) aufweist, der sich an der Stirnseite des Fixierungsstücks abstützt.

9. Vorrichtung nach einem der Ansprüche 6 bis 8, **dadurch gekennzeichnet, dass** der Torsionskörper (3) einen vorspringenden Ansatz (15) aufweist, der in einen seitlichen Schlitz (16) des Fixierungsstücks (9) eingesetzt ist.

10. Vorrichtung nach Anspruch 9, **dadurch gekennzeichnet, dass** der Ansatz (15) des Torsionskörpers (3) als Stellrad ausgebildet ist.

11. Vorrichtung nach einem der Ansprüche 1 bis 10, **dadurch gekennzeichnet, dass** der Basiskörper (2) ein Griffteil (20) aufweist.

12. Vorrichtung nach Anspruch 11, **dadurch gekennzeichnet, dass** der Griffteil (20) zwei parallele Schenkel (18, 19) aufweist, die derart an das Fixierungsstück (9) angesetzt sind, dass durch Zusammendrücken der Schenkel (18, 19) des Griffteils (20) die Schenkel (7a, 7b) des Fixierungsstücks (9) auseinanderspreizbar sind.

13. Vorrichtung nach einem der Ansprüche 1 bis 12, **dadurch gekennzeichnet, dass** eines der Aufnahmestücke (8, 12) des Basiskörpers (2) oder Torsionskörpers (3) oder beide Aufnahmestücke (8, 12) in der Art eines Schnappverschlusses für die Schlauchleitung ausgebildet sind, wobei die Schlauchleitung zwischen zwei Bügeln (29, 30) klemmend fixiert ist.

14. Vorrichtung nach einem der Ansprüche 1 bis 13, **dadurch gekennzeichnet, dass** Basiskörper (2) und Torsionskörper (3) Spritzgießteile aus Kunststoff sind.

15. Überleitsystem zur enteralen Ernährung mit einer Vorrichtung nach einem der Ansprüche 1 bis 14.
